(19)

Europäisches
Patentamt
European
Patent Office
Office européen
des brevets

(11) **EP 3 391 383 B1**

(12) **EUROPEAN PATENT SPECIFICATION**

(45) Date of publication and mention
of the grant of the patent:
**01.12.2021  Bulletin 2021/48**

(51) Int Cl.:
**G21K 4/00** *(2006.01)*        **G01T 1/203** *(2006.01)*

(21) Application number: **16760838.9**

(22) Date of filing: **04.08.2016**

(86) International application number:
**PCT/US2016/045501**

(87) International publication number:
**WO 2017/105556 (22.06.2017 Gazette 2017/25)**

(54) **RADIATION SENSING THERMOPLASTIC COMPOSITE PANELS**

STRAHLUNGSEMPFINDLICHE THERMOPLASTISCHE VERBUNDPLATTEN

PANNEAUX COMPOSITES THERMOPLASTIQUES DE DÉTECTION DE RAYONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.12.2015  US 201562266860 P
08.03.2016  US 201662304970 P**

(43) Date of publication of application:
**24.10.2018  Bulletin 2018/43**

(73) Proprietor: **Carestream Dental Technology Topco
Limited
London SW1Y 6RJ (GB)**

(72) Inventors:
• **JAGANNATHAN, Seshadri
Rochester, NY 14608 (US)**
• **RANKIN, Charles, M.
Rochester, NY 14608 (US)**
• **FOLTS, Lawrence, D.
Rochester, NY 14608 (US)**
• **ULREICH, Barbara
Atlanta, Georgia 30339 (US)**
• **GUFFEY, Betsy, J.
Rochester, NY 14608 (US)**
• **INGLESE, Jean-Marc
Rochester, NY 14608 (US)**

(74) Representative: **Wimmer, Hubert
Wagner & Geyer Partnerschaft mbB
Patent- und Rechtsanwälte
Gewürzmühlstrasse 5
80538 München (DE)**

(56) References cited:
**US-A1- 2006 202 134    US-A1- 2014 291 528**

**Description**

**FIELD OF THE INVENTION**

The invention relates generally to the field of inorganic storage phosphor materials. More specifically, the invention relates to melt extrudable and/or injection moldable and/or hot-melt pressable composites of inorganic storage phosphor materials and thermoplastic and/or thermoset polymers and methods for making the same.

**BACKGROUND OF THE INVENTION**

Near the beginning of the 20th century, it was recognized that a medically useful anatomical image could be obtained when a film containing a radiation-sensitive silver halide emulsion is exposed to X-radiation (X-rays) passing through the patient. Subsequently, it was recognized that X-ray exposure could be decreased considerably by placing a radiographic phosphor panel adjacent to the film.

A radiographic phosphor panel typically contains a layer of an inorganic phosphor that can absorb X-rays and emit light to expose the film. The inorganic phosphor layer is generally a crystalline material that responds to X-rays in an image-wise fashion. Radiographic phosphor panels can be classified, based on the type of phosphors used, as prompt emission panels and image storage panels.

Image storage panels (also commonly referred to as "storage phosphor panels") typically contain a storage ("stimulable") phosphor capable of absorbing X-rays and storing its energy until subsequently stimulated to emit light in an image-wise fashion as a function of the stored X-ray pattern. A well-known use for storage phosphor panels is in computed or digital radiography. In these applications, the panel is first image-wise exposed to X-rays, which are absorbed by the inorganic phosphor particles, to create a latent image. While the phosphor particles may fluoresce to some degree, most of the absorbed X-rays are stored therein. At some interval after initial X-ray exposure, the storage phosphor panel is subjected to longer wave length radiation, such as visible or infrared light (e.g., stimulating light), resulting in the emission of the energy stored in the phosphor particles as stimulated luminescence (e.g., stimulated light) that is detected and converted into sequential electrical signals which are processed in order to render a visible image on recording materials, such as light-sensitive films or digital display devices (e.g., television or computer monitors). For example, a storage phosphor panel can be image-wise exposed to X-rays and subsequently stimulated by a laser having a red light or infrared beam, resulting in green or blue light emission that is detected and converted to electrical signals which are processed to render a visible image on a computer monitor. The stimulating light may also be other sources other than a laser (such as LED lamps), that would permit stimulation of a larger area of the storage phosphor, and the detection may be done using a two dimensional detector, such as a CCD or a CMOS device. Thereafter, images from storage phosphor panels can be "erased" by exposure to UV radiation, such as from fluorescent lamps.

Thus, storage phosphor panels are typically expected to store as much incident X-rays as possible while emitting stored energy in a negligible amount until after subsequent stimulation; only after being subjected to stimulating light should the stored energy be released. In this way, storage phosphor panels can be repeatedly used to store and transmit radiation images.

Reference is also made to US 2014 / 291 528 A1 disclosing radiation sensing thermoplastic composite panels including an extruded inorganic storage phosphor layer including a thermoplastic polyolefin and an inorganic storage phosphor material, wherein the extruded inorganic storage phosphor panel has a DQE comparable to that of a traditional extruded inorganic solvent coated inorganic storage phosphor screen. Also disclosed is an inorganic storage phosphor detection system and a method of making an extruded inorganic storage phosphor panel.

Moreover, US 2006 202 134 A1 discloses a radiation image storage panel having a phosphor layer containing an energy-storing phosphor, in which the storage panel has a surface showing an absorbance of 0.2 to 0.5 at the stimulating wavelength of the phosphor, and the phosphor layer containing the phosphor at a packing density of 3.0 g/cm$^3$ or more.

Thus, there exists a need for improved storage phosphor panels. More specifically, there exists a need for melt extruded or injection molded or hot pressed inorganic storage phosphor panel has an image quality that is comparable to the image quality of the traditional solvent coated screen of equivalent x-ray absorbance.

**SUMMARY OF THE INVENTION**

In accordance with the present invention, a freestanding inorganic storage phosphor panel and a method for manufacturing a freestanding inorganic storage phosphor panel as set forth in Claims 1 and 9, respectively, are provided. Further embodiments of the invention are inter alia disclosed in the dependent claims. An aspect of this application is to advance the art of medical, dental and non-destructive imaging systems.

Another aspect of this application is to address in whole or in part, at least the foregoing and other deficiencies in the related art.

It is another aspect of this application to provide in whole or in part, at least the advantages described herein.

In an aspect, not according to the invention, there are provided exemplary melt extruded or injection molded or hot pressed inorganic storage phosphor panel embodiments including a melt extruded or injection molded or hot pressed inorganic storage phosphor layer comprising a thermoplastic polymer and an inorganic storage phosphor material, wherein the melt extruded or injection molded or hot pressed inorganic storage phosphor panel has an image quality that is comparable to or better than the image quality of the traditional solvent coated screen of equivalent x-ray absorbance.

In another aspect, not according to the invention, there are also disclosed exemplary inorganic storage phosphor detection systems including a melt extruded or injection molded or hot pressed inorganic storage phosphor panel comprising a melt extruded or injection molded or hot pressed inorganic storage phosphor layer comprising a thermoplastic olefin and an inorganic storage phosphor material.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

**BRIEF DESCRIPTION OF THE DRAWINGS**

The foregoing and other objects, features, and advantages of the invention will be apparent from the following more particular description of the embodiments of the invention, as illustrated in the accompanying drawings. The elements of the drawings are not necessarily to scale relative to each other.

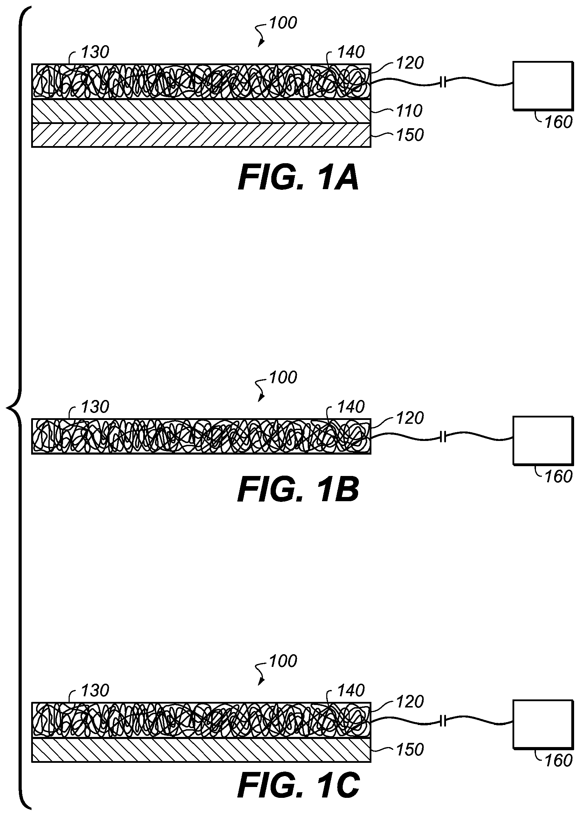

FIGS. 1A-1C depicts exemplary portions of scintillator panels in accordance with various embodiments of the present disclosure.

**DESCRIPTION OF EXEMPLARY EMBODIMENTS**

The following is a description of exemplary embodiments, reference being made to the drawings in which the same reference numerals identify the same elements of structure in each of the several figures.

Exemplary embodiments herein provide free standing inorganic storage phosphor panels comprising the technical features of claim 1, in particular including an extruded storage phosphor layer with a thermoplastic polymer and a storage phosphor material, and methods for manufacturing thereof. It should be noted that while the present description and examples are primarily directed to radiographic medical imaging of a human or other subject, embodiments of apparatus and methods of the present application can also be applied to other radiographic imaging applications. This includes applications such as non-destructive testing (NDT), for which radiographic images may be obtained and provided with different processing treatments in order to accentuate different features of the imaged subject.

An important property of the screen is its x-ray absorbance. Depending on the specific application (orthopedic or mammography or intra- oral dental or extra oral dental or non-destructive testing of metals or...), the energy and the intensity of the radiation that is incident on the storage phosphor screen will be different. However, in order to be value as an x-ray imaging tool, the storage phosphor screen has to have sufficient x-ray absorbance, so as to produce a useful image. In practical terms, this requires that ~ 40-60% of the extruded storage phosphor screen (by volume) be the storage phosphor material (barium fluorobromoiodide or cesium bromide).

Another requirement for the storage phosphor screen is that it be readable from either side of the screen, and in the transmission or the reflection mode, with respect to the direction of incidence of the stimulation radiation used for reading the information in the screen. And it would desirable that the screen can be handled under ambient lighting conditions or room light.

Depending on the specific imaging application (medical radiography or dental radiography or non-destructive testing), the physical characteristics required of the storage phosphor panel can be widely different. However, the divergent physical properties may be defined by a few key properties of the storage phosphor screen, such as its bending resistance (http://www.taberindustries.com/stiffness-tester), tear resistance (http://jlwinstruments.com/index.php/products/test-solutions/tear-resistance-testing/) or folding resistance (https://www.testingmachines.com/product/31-23-mit-folding-endurance-tester). A summary of various methods to measure these properties is outlined in (http://ipst.gatech.edu/faculty/popil_roman/pdf presentations/Prediction%20of%20Fold%20Crackins%20Propensity%20through%20Physical%20Testing.pdf). All this may be achieved using a single layer or a multi layered architecture, that would include additional, coextruded layers on the screen, that may contain particulates and/or chemistry to achieve the required physical properties needed to accommodate the mechanics of the scanner and/or handling by the end user. Further, it is important that the extruded storage phosphor screen be recyclable; i.e., it is necessary that the composition of the screen is such that they can re-used to make the storage phosphor screen, and/or the storage phosphor part of the screen can be reused to manufacture a new screen.

The stimulation wavelength and the emission wavelength of the storage phosphor panel are generally determined by the specific storage phosphor. The peak stimulation wavelength for the commonly used storage phosphors, the stimulation wavelength is fairly broad, and is in the region of 550-700 nm. However, the stimulated emission for the europium doped barium fluorobromoiodide storage phosphor has peak around 390 nm.

FIG. 1 depicts a portion of an exemplary storage phosphor panel 100 in accordance with various embodiments of the present disclosure. As used herein, "storage phosphor panel" is understood to have its ordinary meaning in the art unless otherwise specified, and refers to panels or screens that store the image upon exposure to X-radiation and emit light when stimulated by another (generally visible) radiation. As such, "panels" and "screens" are used interchangeably herein. It should be readily apparent to one of ordinary skill in the art that the storage phosphor panel 100 depicted in FIGS. 1A-1C represents a generalized schematic illustration and that other components can be added or existing components can be removed or modified.

Storage phosphor panels disclosed herein can take any convenient form provided they meet all of the usual requirements for use in computed radiography. As shown in FIG. 1A, the storage phosphor panel 100 may include a support 110 and a melt extruded or injection molded or hot pressed storage phosphor layer 120 disposed over the support 110. Any flexible or rigid material suitable for use in storage phosphor panels and does not interfere with the recyclability of storage phosphor screen can be used as the support 110, such as glass, plastic films, ceramics, polymeric materials, carbon substrates, and the like. In certain embodiments, the support 110 can be made of ceramic, (*e.g.*, $Al_2O_3$) or metallic (e.g., Al) or polymeric (e.g., polypropylene) materials. Also as shown in FIG. 1A, in an aspect, the support 110 can be coextruded with the storage phosphor layer 120. The support may be transparent, translucent, opaque, or colored (e.g., containing a blue or a black dye). Alternatively, if desired, a support can be omitted in the storage phosphor panel.

In another aspect, an anticurl layer may be coextruded on either side of the support, if a support is used, or on side of the storage phosphor screen, to manage the dimensional stability of the storage phosphor screen.

The thickness of the support 110 can vary depending on the materials used so long as it is capable of supporting itself and layers disposed thereupon. Generally, the support can have a thickness ranging from about 50 $\mu$m to about 1,000 $\mu$m, for example from about 80 $\mu$m to about 1000 $\mu$m, such as from about 80 $\mu$m to about 500 $\mu$m. The support 110 can have a smooth or rough surface, depending on the desired application. In an embodiment, the storage phosphor panel does not comprise a support.

The storage phosphor layer 120 can be disposed over the support 110, if a support is included. Alternatively, the storage phosphor layer 120 can be melt extruded or injection molded or hot pressed independently as shown in FIG. 1B, or melt extruded or injection molded or hot pressed together with an opaque layer, and anticurl layer, and combinations thereof, e.g., shown as layer 150, in FIG. 1A and FIG. 1C.

The storage phosphor layer 120 can include a thermoplastic polymer 130 and a storage phosphor material 140. The thermoplastic polymer 130 may be a polyolefin, such as polyethylene, a polypropylene, and combinations thereof, or a polyurethane, a polyester, a polycarbonate, a silicone, a siloxane, a polyvinyl chloride (PVC), a polyvinylidine chloride (PVdC). In an aspect, the polyethylene can be high density poly low density polyethylene (LDPE), medium density polyethylene (MDPE), linear low density polyethylene (LLDPE), very low density polyethylene (VLDPE), and the like. In a preferred embodiment, the thermoplastic polymer 130 is low density polyethylene (LDPE). The thermoplastic polymer 130 can be present in the storage phosphor layer 120 in an amount ranging from about 1% to about 50% by volume, for example from about 10% to about 30% by volume, relative to the total volume of the storage phosphor layer 120.

As used herein, "storage phosphor particles" and "stimulable phosphor particles" are used interchangeably and are understood to have the ordinary meaning as understood by those skilled in the art unless otherwise specified. "Storage phosphor particles" or "stimulable phosphor particles" refer to phosphor crystals capable of absorbing and storing X-rays and emitting electromagnetic radiation (e.g., light) of a second wavelength when exposed to or stimulated by radiation of still another wavelength. Generally, stimulable phosphor particles are turbid polycrystals having particle diameters of several micrometers to several hundreds of micrometers; however, fine phosphor particles of submicron to nano sizes have also been synthesized and can be useful. Thus, the optimum mean particle size for a given application is a reflection of the balance between imaging speed and desired image sharpness.

Stimulable phosphor particles can be obtained by doping, for example, rare earth ions as an activator into a parent material such as oxides, nitrides, oxynitrides, sulfides, oxysulfides, silicates, halides, and the like, and combinations thereof. As used herein, "rare earth" refers to chemical elements having an atomic number of 39 or 57 through 71 (also known as "lanthanoids"). Stimulable phosphor particles are capable of absorbing a wide range of electromagnetic radiation. In exemplary preferred embodiments, stimulable phosphor particles can absorb radiation having a wavelength of from about 0.01 to about 10 nm (e.g., X-rays) and from about 300 nm to about 1400 nm (e.g., UV, visible, and infrared light). When stimulated with stimulating light having a wavelength in the range of visible and infrared light, stimulable phosphor particles can emit stimulated light at a wavelength of from about 300 nm to about 650 nm.

Suitable exemplary stimulable phosphor particles for use herein include, but are not limited to, compounds having Formula (I):

$$MFX_{1-z}I_zuM^aX^a:yA:eQ:tD \qquad (I)$$

wherein M is selected from the group consisting of Mg, Ca, Sr, Ba, and combinations thereof;

X is selected from the group consisting Cl, Br, and combinations thereof;
$M^a$ is selected from the group consisting of Na, K, Rb, Cs, and combinations thereof;
$X^a$ is selected from the group consisting of F, Cl, Br, I, and combinations thereof;
A is selected from the group consisting of Eu, Ce, Sm, Th, Bi, and combinations thereof;
Q is selected from the group consisting of BeO, MgO, CaO, SrO, BaO, ZnO, $Al_2O_3$, $La_2O_3$, $In_2O_3$, $SiO_2$, $TiO_2$, $ZrO_2$, $GeO_2$, $Nb_2O_5$, $Ta_2O_5$, $ThO_2$, and combinations thereof;
D is selected from the group consisting of V, Cr, Mn, Fe, Co, Ni, and combinations thereof;
z is from about 0.0001 to about 1;
u is from about 0 to about 1;
y is from about 0.0001 to about 0.1;
e is from 0 to about 1; and
t is from 0 to about 0.01.

The amounts represented by "z", "u", "y", "e", and "t" are molar amounts. The same designations appearing elsewhere in this disclosure have the same meanings unless otherwise specified. In Formula (I), preferably, M is Ba; X is Br; $M^a$ is selected from the group consisting of Na, K, and combinations thereof; $X^a$ is selected from the group consisting of F, Br, and combinations thereof; A is Eu; Q is selected from the group consisting of $SiO_2$, $Al_2O_3$, and combinations thereof; and t is 0.

Other exemplary stimulable phosphor particles for use herein include, but are not limited to, compounds having Formula (II):

$$(Ba_{1-a-b-c}Mg_aCa_bSr_c)FX_{1-z}I_zrM^aX^a:yA:eQ:tD \qquad (II)$$

wherein X, $M^a$, $X^a$, A, Q, D e, t, z, and y are as defined above for Formula (I); the sum of a, b, and c, is from 0 to about 0.4; and r is from about $10^{-6}$ to about 0.1.

In Formula (II), preferably X is Br; $M^a$ is selected from the group consisting of Na, K, and combinations thereof; $X^a$ is selected from the group consisting of F, Br, and combinations thereof; A is selected from the group consisting of Eu, Ce, Bi, and combinations thereof; Q is selected from the group consisting of $SiO_2$, $Al_2O_3$, and combinations thereof; and t is 0.

Further exemplary stimulable phosphor particles for use herein include, but are not limited to, compounds having Formula (III):

$$M^{1+}X_aM^{2+}X'_2bM^{3+}X''3:cZ \qquad (III)$$

wherein M is selected from the group consisting of Li, na, K, Cs, Rb, and combinations thereof;

$M^{2+}$ is selected from the group consisting of Be, Mg, Ca, Sr, Ba, Zn, Cd, Cu, Pb, Ni, and combinations thereof;
$M^{3+}$ is selected from the group consisting of Sc, Y, La, Ce, Pr, Nd, Pm, Sm, Eu, Gd, Tb, Dy Ho, Er, Tm Yb, Lum Al, Bi, In, Ga, and combinations thereof;
Z is selected from the group consisting of $Ga^{1+}$, $Ge^{2+}$, $Sn^{2+}$, $Sb^{3+}$, $As^{3+}$, and combinations thereof;
X, X' and X" can be the same or different and each individually represents a halogen atom selected from the group consisting of F, Br, Cl, I; and $0 \leq a \leq 1$; $0 \leq b \leq 1$; $0 < c \leq 0.2$.

Preferred stimulable phosphor particles represented by Formulas (I) , (II), or (III) include europium activated barium fluorobromides (e.g., BaFBr:Eu and BaFBrI:Eu), cerium activated alkaline earth metal halides, cerium activated oxyhalides, divalent europium activated alkaline earth metal fluorohalides, (e.g., Ba(Sr)FBr:$Eu^{2+}$) divalent europium activated alkaline earth metal halides, rare earth element activated rare earth oxyhalides, bismuth activated alkaline earth metal halide phosphors, and combinations thereof.

An alternative to the Eu doped BaFBrI type storage phosphor is, Eu doped CsBr storage phosphor. This is generally used in the form a binderless storage phosphor screen, where the needle shaped Eu doped CsBr particles are generated by vapor deposition of the material on a substrate, which is then sealed water impermeable material. Such needle shaped europium doped cesium bromide storage phosphor screen has an emission peak around 450 nm.

The thermoplastic polymer and the inorganic storage phosphor material are melt compounded to form composite thermoplastic particles which are then melt extruded or injection molded or hot pressed to form the inorganic storage phosphor layer. For example, the composite thermoplastic particles can be prepared by melt compounding the thermoplastic polymer with the inorganic storage phosphor material using a twin screw compounder. The ratio of thermoplastic polymer to inorganic storage phosphor material (polymer: inorganic storage phosphor) can range from about 1:100 to about 1:0.01, by weight or volume, preferably from about 1:1 to about 1:0.1, by weight or volume. The composition may include inorganic, organic and/or polymeric additives to manage image quality and/or the physical properties of the extruded storage phosphor screen. Examples of the additives include, a blue dye (e.g., ultramarine blue, copper phthalocyanine,..) for managing image quality, surfactants (e.g., sodium dodecyl sulfate) for managing the colloidal stability of the storage phosphor particles, polymers (e.g., ethylene vinylacetate) for managing the rheology of the composite. During melt compounding, the thermoplastic polymer and the inorganic storage phosphor material can be compounded and heated through multiple heating zones. For example, in the case of polyolefins, the temperature of the heating zones can vary from ca. 170 $^0$C — 250 $^0$C, depending on the specific composition of the polymer/additive blends that are used, and the period of time in each zone depends on the polymer used and the temperature of the heating zone. Generally, the polymer can be heated for a time and temperature sufficient to melt the polymer and incorporate the inorganic storage phosphor material without decomposing the polymer. The period of time in each zone can range from about 1 second to about 1 minute. Upon exiting the melt compounder, the composite thermoplastic material can enter a water bath to cool and harden into continuous strands. The strands can be pelletized and dried at about 40°C. The screw speed and feed rates for each of the thermoplastic polymer 130 and the inorganic storage phosphor material 140 can be adjusted as desired to control the amount of each in the composite thermoplastic material.

Alternatives to melt compounding not part of the present invention include the creation of the composite mixture in an appropriate solvent where the polymer is dissolved or dispersed and inorganic storage phosphor particles are dispersed, followed by the evaporation of the solvent and the milling of the polymer/inorganic storage phosphor composite mixture is pelletized using grinders, cryo-grinder, densifiers, agglomerators, or any other suitable device.

The inorganic storage phosphor/thermoplastic polymer composite material can be melt extruded or injection molded or hot pressed to form the inorganic storage phosphor layer in which the inorganic storage phosphor material is intercalated ("loaded") within the thermoplastic polymer. For example, the inorganic storage phosphor/thermoplastic polymer composite layer can be formed by melt extruding or injection molding or hot pressing the composite thermoplastic material. Without being limited by theory, it is believed that forming the inorganic storage phosphor/thermoplastic composite layer by melt extrusion or injection molding or hot pressing increases the homogeneity of the inorganic storage phosphor layer, and eliminates the undesirable "evaporated space" generated when the solvent is evaporated in the traditional solvent-coated panels. A melt extruded or injection molded or hot pressed inorganic storage phosphor/thermoplastic composite panel according to the present disclosure can have comparable image quality, as compared to the traditional solvent coated panels, along with improved mechanical and environmental robustness.

In the case of the inorganic storage phosphor/thermoplastic polymer composite layer being melt extruded or injection molded or hot pressed in combination with a support layer, the melt processing parameters (temperature, screw speed and pump speed in the case of melt extrusion and injection molding, and temperature and pressure in the case of hot pressing) can be adjusted to control the thickness for each of the inorganic storage phosphor/thermoplastic polymer composite layer and the support layer, individually.

The thickness of the inorganic storage phosphor /thermoplastic composite layer can range from about 10 $\mu$m to about 1000 $\mu$m, preferably from about 50 $\mu$m to about 750 $\mu$m, more preferably from about 100 $\mu$m to about 500 $\mu$m.

Optionally, the melt extruded or injection molded or hot pressed inorganic storage phosphor panel can include a protective overcoat disposed over the inorganic storage phosphor/thermoplastic composite layer, which provides enhanced mechanical strength and scratch and moisture resistance, if desired.

In an embodiment, a scintillation detection system can include the disclosed storage phosphor panel 100 coupled, inserted or mounted to at least one storage phosphor panel reader/scanner 160. Choice of a particular storage phosphor reader will depend, in part, on the type of storage phosphor panel being fabricated and the intended use of the ultimate device used with the disclosed storage phosphor panel.

ASSESSMENT

An important characteristic of the melt extruded or injection molded or hot pressed inorganic storage phosphor panel is that it can be recycled and reused by simply grinding (e.g., mechanically or electromechanically) the panels using a standard device such as a grinder, cryo-grinder, densifiers, agglomerators, or any other suitable device, and pelletizing it during the crushing process, and/or subsequently by a compounding processes described above, and extruding it again as a storage phosphor panel. In order to do be able to recycle the melt extruded or injection molded or hot pressed inorganic storage phosphor panel effectively, it is necessary that the storage phosphor panel can be ground without abrading the grinding equipment. An important measure of the ability to grind the melt extruded or injection molded or hot pressed inorganic storage phosphor panel panels without abrading the grinding equipment is its fracture strength of the panel. It is necessary that the fracture strength of the melt extruded or injection molded or hot pressed inorganic storage phosphor panel panels is as small as possible, while being high enough to sustain routine handling during use.

Thus, it is useful to compare grinding or tearing parameters for inventive and conventional inorganic storage phosphor panels. For practical purposes, it is useful to compare the peak torque required (e.g., used) to tear inorganic storage phosphor panels, using a standard or conventional method and equipment, such as the SER (Sentmanat Extension Rheometer) Universal Testing Platform from Xpansion Instruments (http://www.xinst.com/products.htm). One of the modes of operation of the instrument is the "high rate fracture testing", which is described in the website (http://www.xinst.com/results fracture.htm). The operational details of the instrument are described in http://www.xinst.com/images/SER_2004 Antec.pdf.

The fracture of the films measured by this device is defined by the following equation.

$$FS = T_p/2Rt$$

Where FS - Fracture strength of the film being measured (Newtons/meter),
Tp - the peak torque applied by the instrument (Newtons meter), R - radius of the drum (meters), and t - thickness of the film (meters)

The peak torque (Tp) required for tearing the panel is described the following equation

$$Tp = FS \times 2 \times R \times t$$

The image quality assessments were done as described below. The x-ray source was a Carestream Health CS2200 x-ray generator and the images were scanned using a Carestream Health CS7600 intra oral dental scanner, in the super high resolution mode. The screens were subjected to an x-ray exposure of 70kV, 7 mA, 0.16 sec. The pixel values were obtained using a flat field exposure of the storage phosphor screen and the resolution was obtained by imaging a line pair phantom, and visual observation. Image quality assessments obtained herein provide a measureable line pairs per mm (LP/mm) resolution consistently or across a set or batch of manufactured panels (e.g., an average resolution). For a constant x-ray exposure, fixed scanner conditions, the linearized pixel code value (cv) represents the speed of the storage phosphor screen in converting the incident x-ray photons to optical photons by photostimulated luminescence, which is detected by the detector and converted into digital signals.

## COMPARATIVE EXAMPLE 1

A solvent coated intraoral computed radiography panels (CS7600) was used as a comparative example. The panel consists of a BaFBrI phosphor coated on 7 mil PET support, and has a ~ 6 micron PVDF/PMMA overcoat, and the edges are sealed using the same overcoat material. Solvent coated intraoral computed radiography panels currently use a PET support. Applicants further were unable to tear an isolated 6 mil PET support, which could be used as the support for an intraoral computed radiography panel. The total thickness was 325 microns.

## INVENTIVE EXAMPLE 1

COMPOSITE THERMOPLASTIC PARTICLE PRODUCTION

Inorganic storage phosphor/thermoplastic composite pellets according to the present disclosure were prepared comprising 86 % wt. barium flurobromoiodide (BFBrI) and 14% wt low density polyethylene (LDPE EM811A, available from Westlake Longview Corp. of Houston, TX), contained copper phthalocyanine, a blue dye, at a level of approximately 200 ppm with respect to the phosphor.

The formulation involved blending and compounding of the EM811A polymer resin with the the BFBrI powder using a Leistritz GL-400 twin screw compounder. The screw speed was set at 300 RPM. The die temperature was set to 220°C and 10 heating zones within the compounder were set to the temperatures shown in Table 3 below:

TABLE 1

| Zone | 1 | 2 | 3 | 4 | 5 | 6 | 7 | 8 | 9 | 10 |
|---|---|---|---|---|---|---|---|---|---|---|
| Temp (°C) | 220 | 220 | 220 | 220 | 220 | 220 | 220 | 220 | 220 | 220 |

After exiting the die, the inorganic storage phosphor/thermoplastic composite pellets, comprising LDPE loaded with BFBrI, entered a 25°C water bath to cool and hardened into continuous strands. The strands were then fed into a pelletizer and dried at 40 °C.

EXTRUSION OF INORGANIC STORAGE PHOSPHOR LAYER

The pelletized composite thermoplastic materials were loaded into a single screw Davis Standard extruder. Within the extruder, heating zones were set to the temperatures shown in Table 2.

TABLE 2

| Davis Standard Extruder | |
|---|---|
| Zone | Temp |
| 1 | 390°F |
| 2 | 420°F |
| 3 | 430°F |
| 4 | 430°F |
| Gate | 430°F |
| Die | 430°F |

The pelletized material (composite thermoplastic) was extruded through a single die with the die temperature set at 430°F form an extruded inorganic storage phosphor panel in the thickness range of 100-200 microns. The total thickness was 150 microns.

**INVENTIVE EXAMPLE 2**

COMPOSITE THERMOPLASTIC PARTICLE PRODUCTION

A sample was prepared as described in inventive example 1, with a primary difference being that the pelletized material (composite thermoplastic) was coextruded through a two slot die with a carbon black containing low density polyethylene (LDPE EM811A, available from Westlake Longview Corp. of Houston, TX), with the die temperature set at 430°F, to form a bilayer extruded inorganic storage phosphor panel in the thickness range of 300-350 microns, with a carbon black containing LDPE layer. The blue dye concentration in the phosphor layer was 100 ppm with respect to the phosphor, and the total thickness was 325 microns.

**ANALYSIS**

| Sample | Support Thickness (microns) | Phosphor layer Thickness (microns) | Screen x-ray absorbance | Screen speed (linearized pixel CV) | (speed/ x-ray absorbance) | Screen resolution (LP/mm) | Peak torque (Tp) in Newton meter |
|---|---|---|---|---|---|---|---|
| Comparative Example 1 | 175 | 150 | 0.259 | 3556 | $1.37 \times 10^4$ | 17 | Could not be torn (maximum torque that can applied by the instrument is 0.2) |
| Inventive Example 1 | none | 150 | 0.203 | 2989 | $1.47 \times 10^4$ | 18 | 0.048 |
| Inventive Example 2 | 175 | 150 | 0.232 | 3186 | $1.37 \times 10^4$ | 18 | 0.102 |

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim.

Certain exemplary method and/or apparatus embodiments according to the application can provide virtual definition of the base of a dental virtual model. Exemplary embodiments according to the application can include various features described herein (individually or in combination).

While the invention has been illustrated with respect to one or more implementations, alterations and/or modifications can be made to the illustrated examples without departing from the scope of the appended claims. The term "at least one of' is used to mean one or more of the listed items can be selected. The term "about" indicates that the value listed can be somewhat altered, as long as the alteration does not result in nonconformance of the process or structure to the illustrated embodiment. Further, "exemplary" indicates the description is used as an example, rather than implying that it is an ideal. Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A free standing inorganic storage phosphor panel (100) comprising:

   an inorganic storage phosphor layer (120) comprising at least one thermoplastic polymer (130), an inorganic storage phosphor material (140) and a copper phthalocyanine blue dye,
   wherein the inorganic storage phosphor layer (120) is formed by melt compounding the at least one thermoplastic polymer (130), inorganic storage phosphor material (140) and copper phthalocyanine blue dye to form a melt compounded material and by then melt extruding, injection molding, or hot pressing the melt compounded material;
   wherein the thermoplastic polymer (130) is a polyolefin, a polypropylene, a polyurethane, a polyester, a polycarbonate, a silicone, a siloxane, a polyvinyl chloride or a polyvinylidine chloride; and
   wherein a peak torque (Tp) required for tearing the panel is greater than 0.04 Newton meter and less than 0.2 Newton meter.

2. The free standing inorganic storage phosphor panel (100) of claim 1, wherein the inorganic storage phosphor material (140) comprises particles in which 95% of the particles are less than or equal to 6.8 microns in diameter and 95% of the particles are greater than or equal to 1.0 microns in diameter.

3. The free standing inorganic storage phosphor panel (100) of claim 1, further comprising a support (110) comprising at least one polymer with or without a light absorbing material.

4. The free standing inorganic storage phosphor panel (100) of claim 1, wherein the at least one thermoplastic polyolefin is a polyethylene.

5. The free standing inorganic storage phosphor panel (100) of claim 1, wherein the panel (100) has a resolution greater than or equal to 15 line pairs per millimeter (lp/mm).

6. The free standing inorganic storage phosphor panel (100) of claim 1, wherein the polyolefin is polyethylene, a polypropylene or combinations thereof.

7. The free standing inorganic storage phosphor panel (100) of claim 7, wherein the polyethylene is a high density poly low density polyethylene, a medium density polyethylene, a linear low density polyethylene, or a very low density polyethylene.

8. The free standing inorganic storage phosphor panel (100) of claim 8, wherein the thermoplastic polymer (130) is a low density polyethylene which is present in the storage phosphor layer (120) in an amount ranging from about 1% to about vol. 50%.

9. A method for manufacturing a free standing inorganic storage phosphor panel (100) according to anyone of the preceding claims, the method comprising:

melt compounding an inorganic storage phosphor layer (120) including at least one thermoplastic polymer (130), an inorganic storage phosphor material (140) and a copper phthalocyanine blue dye to produce a melt compounded material;

melt extruding, injection molding, or hot pressing the melt compounded material to form a free standing manufactured inorganic storage phosphor panel (100);

wherein the thermoplastic polymer (130) is a polyolefin, a polypropylene, a polyurethane, a polyester, a polycarbonate, a silicone, a siloxane, a polyvinyl chloride or a polyvinylidine chloride; and

wherein a peak torque (Tp) required for tearing the panel is greater than 0.04 Newton meter and less than 0.2 Newton meter.

10. The method of claim 9, wherein the at least one thermoplastic polymer (130) is a thermoplastic polyolefin, and wherein the inorganic storage phosphor material (140) comprises particles in which 95% of the particles are less than or equal to 6.8 microns in diameter and 95% the particles are greater than or equal to 1.0 microns in diameter.

**Patentansprüche**

1. Eine freistehende, anorganische Speicher-Phosphor- bzw. Leuchtstoffplatte (100), die Folgendes aufweist:

eine anorganische Speicher-Phosphor- bzw. Leuchtstoffschicht (120), die mindestens ein thermoplastisches Polymer (130), ein anorganisches Speicher-Phosphor- bzw. Leuchtstoffmaterial (140) und einen blauen Kupferphthalocyanin-Farbstoff aufweist,

wobei die anorganische Speicher-Leuchtstoffschicht (120) gebildet wird durch Schmelzcompoundieren des mindestens einen thermoplastischen Polymers (130), des anorganischen Speicher-Leuchtstoffmaterials (140) und des blauen Kupferphthalocyanin-Farbstoffs, um ein schmelzcompoundiertes Material zu bilden, und durch anschließendes Schmelzextrudieren,

Spritzgießen oder Heißpressen des schmelzcompoundierten Materials;

wobei das thermoplastische Polymer (130) ein Polyolefin, ein Polypropylen, ein Polyurethan, ein Polyester, ein Polycarbonat, ein Silikon, ein Siloxan, ein Polyvinylchlorid oder ein Polyvinylidinchlorid ist; und

wobei ein zum Zerreißen der Platte erforderliches Spitzendrehmoment (Tp) größer als 0,04 Newtonmeter und kleiner als 0,2 Newtonmeter ist.

2. Die freistehende, anorganische Speicher-Leuchtstoffplatte (100) nach Anspruch 1, wobei das anorganische Speicher-Leuchtstoffmaterial (140) Partikel aufweist, bei denen 95 % der Partikel einen Durchmesser von höchstens 6,8 Mikrometern und 95 % der Partikel einen Durchmesser von höchstens 1,0 Mikrometern aufweisen.

3. Die freistehende, anorganische Speicher-Leuchtstoffplatte (100) nach Anspruch 1, die ferner einen Träger (110) aufweist, der mindestens ein Polymer mit oder ohne einem lichtabsorbierenden Material aufweist.

4. Die freistehende, anorganische Speicher-Leuchtstoffplatte (100) nach Anspruch 1, wobei das mindestens eine thermoplastische Polyolefin ein Polyethylen ist.

5. Die freistehende, anorganische Speicher-Leuchtstoffplatte (100) nach Anspruch 1, wobei die Platte (100) eine Auflösung von mindestens 15 Linienpaaren pro Millimeter (lp/mm) aufweist.

6. Die freistehende, anorganische Speicher-Leuchtstoffplatte (100) nach Anspruch 1, wobei das Polyolefin Polyethylen ist, ein Polypropylen oder Kombinationen davon.

7. Die freistehende, anorganische Speicher-Leuchtstoffplatte (100) nach Anspruch 6, wobei das Polyethylen ein Hochdichte-Polyniedrigdichte-Polyethylen, ein Polyethylen mit mittlerer Dichte, ein lineares Polyethylen mit niedriger Dichte oder ein Polyethylen mit sehr niedriger Dichte ist.

8. Die freistehende, anorganische Speicher-Leuchtstoffplatte (100) nach Anspruch 7, wobei das thermoplastische Polymer (130) ein Polyethylen mit niedriger Dichte ist, das in der Speicher-Leuchtstoffschicht (120) in einer Menge von etwa 1 % bis etwa 50 % vorhanden ist.

**9.** Ein Verfahren zur Herstellung einer freistehenden, anorganischen Speicher-Leuchtstoffplatte (100) nach einem der vorhergehenden Ansprüche, wobei das Verfahren Folgendes aufweist:

Schmelzcompoundieren einer anorganischen Speicher-Phosphor- bzw. Leuchtstoffschicht (120), die mindestens ein thermoplastisches Polymer (130), ein anorganisches Speicher-Phosphor- bzw. Leuchtstoffmaterial (140) und einen Kupferphthalocyaninblau-Farbstoff enthält, um ein schmelzcompoundiertes Material herzustellen;

Schmelzextrudieren, Spritzgießen oder Heißpressen des schmelzcompoundierten Materials zum Bilden einer freistehend hergestellten, anorganischen Speicher-Leuchtstoffplatte (100);

wobei das thermoplastische Polymer (130) ein Polyolefin, ein Polypropylen, ein Polyurethan, ein Polyester, ein Polycarbonat, ein Silikon, ein Siloxan, ein Polyvinylchlorid oder ein Polyvinylidinchlorid ist; und

wobei ein zum Zerreißen der Platte erforderliches Spitzendrehmoment (Tp) größer als 0,04 Newtonmeter und kleiner als 0,2 Newtonmeter ist.

**10.** Das Verfahren nach Anspruch 9, wobei das mindestens eine thermoplastische Polymer (130) ein thermoplastisches Polyolefin ist, und wobei das anorganische Speicher-Leuchtstoffmaterial (140) Partikel aufweist, bei denen 95 % der Partikel einen Durchmesser von höchstens 6,8 Mikrometern und 95 % der Partikel einen Durchmesser von höchstens 1,0 Mikrometern aufweisen.

## Revendications

**1.** Panneau luminophore inorganique de stockage autonome (100) comprenant :

une couche inorganique luminophore de stockage (120) comprenant au moins un polymère thermoplastique (130), un matériau inorganique luminophore de stockage (140) et un colorant bleu de phtalocyanine de cuivre, dans lequel la couche inorganique luminophore de stockage (120) est constituée en formant un composé fondu dudit au moins un polymère thermoplastique (130), du matériau inorganique luminophore de stockage (140) et du colorant bleu de phtalocyanine de cuivre pour former un matériau composite fondu puis en extrudant à chaud, moulant par injection ou pressant à chaud le matériau composite fondu ;

dans lequel le polymère thermoplastique (130) est une polyoléfine, un polypropylène, un polyuréthane, un polyester, un polycarbonate, un silicone, un siloxane, un polychlorure de vinyle ou un polychlorure de vinylidène ; et

dans lequel un couple maximal (Tp) requis pour déchirer le panneau est supérieur à 0,04 Newton mètre et inférieur à 0,2 Newton mètre.

**2.** Panneau luminophore inorganique de stockage autonome (100) selon la revendication 1, dans lequel le matériau inorganique luminophore de stockage (140) comprend des particules dans lesquelles 95 % des particules ont un diamètre inférieur ou égal à 6,8 microns et 95 % des particules ont un diamètre supérieur ou égal à 1,0 micron.

**3.** Panneau luminophore inorganique de stockage autonome (100) selon la revendication 1, comprenant en outre un support (110) comprenant au moins un polymère avec ou sans matériau absorbant la lumière.

**4.** Panneau luminophore inorganique de stockage autonome (100) selon la revendication 1, dans lequel ladite au moins une polyoléfine thermoplastique est un polyéthylène.

**5.** Panneau luminophore inorganique de stockage autonome (100) selon la revendication 1, dans lequel le panneau (100) a une résolution supérieure ou égale à 15 paires de lignes par millimètre (lp/mm).

**6.** Panneau luminophore inorganique de stockage autonome (100) selon la revendication 1, dans lequel la polyoléfine est un polyéthylène, un polypropylène ou des combinaisons de ceux-ci.

**7.** Panneau luminophore inorganique de stockage autonome (100) selon la revendication 6, dans lequel le polyéthylène est un polyéthylène basse densité poly haute densité, un polyéthylène à densité moyenne, un polyéthylène à basse densité linéaire ou un polyéthylène à très basse densité.

**8.** Panneau luminophore inorganique de stockage autonome (100) selon la revendication 7, dans lequel le polymère thermoplastique (130) est un polyéthylène à basse densité qui est présent dans la couche luminophore de stockage (120) dans une quantité se trouvant dans la plage allant d'environ 1 % à environ 50 % en volume.

9. Procédé de fabrication d'un panneau luminophore inorganique de stockage (100) selon l'une quelconque des revendications précédentes, le procédé comprenant :

la formation d'un composé fondu d'une couche inorganique luminophore de stockage (120) comportant au moins un polymère thermoplastique (130), un matériau inorganique luminophore de stockage (140) et un colorant bleu de phtalocyanine de cuivre pour produire un matériau composé fondu ;
l'extrusion à l'état fondu, le moulage par injection ou le pressage à chaud du matériau composite fondu pour former un panneau luminophore inorganique de stockage fabriqué autonome (100) ;
dans lequel le polymère thermoplastique (130) est une polyoléfine, un polypropylène, un polyuréthane, un polyester, un polycarbonate, un silicone, un siloxane, un polychlorure de vinyle ou un polychlorure de vinylidène ; et
dans lequel un couple maximal (Tp) requis pour déchirer le panneau est supérieur à 0,04 Newton mètre et inférieur à 0,2 Newton mètre.

10. Procédé selon la revendication 9, dans lequel le polymère thermoplastique (130) est une polyoléfine thermoplastique, et
dans lequel le matériau inorganique luminophore de stockage (140) comprend des particules dans lesquelles 95 % des particules ont un diamètre inférieur ou égal à 6,8 microns et 95 % des particules ont un diamètre supérieur ou égal à 1,0 micron.

**FIG. 1A**

**FIG. 1B**

**FIG. 1C**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014291528 A1
- US 2006202134 A1